# EUROPEAN PATENT SPECIFICATION

(11) **EP 3 493 879 B1**
(45) Date of publication and mention of the grant of the patent: **21.10.2026**
(21) Application number: 17749029.9
(22) Date of filing: 25.07.2017
(51) Int. Cl.: A61Q 1/04, A61K 8/891, A61K 8/898

(54) **LONG-WEARING, TRANSFER-RESISTANT COSMETIC COMPOSITION HAVING IMPROVED TACKINESS**
LANGANHALTENDE, ÜBERTRAGUNGSFESTE KOSMETISCHE ZUSAMMENSETZUNG MIT VERBESSERTER HAFTUNG
COMPOSITION COSMÉTIQUE A TENUE DE LONGUE DURÉE, RÉSISTANTE AU TRANSFERT, PRÉSENTANT UN POUVOIR ADHÉSIF AMÉLIORÉ

(30) Priority: 29.07.2016 US 201615223746
(43) Date of publication of application: 12.06.2019
(73) Proprietor: L'OREAL, 75008 Paris (FR)
(72) Inventor: ROSARIO-MELENDEZ, Roselin, Clark, NJ 07066 (US); EL-KHOURI, Rita Jaky, Clark, NJ 07066 (US); PERRUNA, Gisela, Clark, NJ 07066 (US); ROHMEYER, Ann Marie, Clark, NJ 07066 (US); DESTENO, Susan Ashley, Clark, NJ 07066 (US)
(74) Representative: ABG Intellectual Property Law, S.L.
(86) International application number: PCT/US2017/043719
(87) International publication number: WO 2018/022614

(56) References cited:
- US-A1- 2007 053 859
- US-A1- 2012 171 138

## Description

### CROSS-REFERENCE TO RELATED APPLICATIONS

This application claims the benefit of priority from U.S. Non-provisional Application No. 15/223,746 filed on July 29, 2016.

### FIELD OF THE INVENTION

The present invention relates to a cosmetic composition and method for making up and/or enhancing the appearance of a keratinous substrate, comprising at least one silicone elastomer, at least one non-volatile oil, at least one silicone resin, at least one volatile solvent and at least one polyorganosiloxane copolymer. The composition of the present invention may optionally contain waxes, fillers and/or pigments.

### BACKGROUND OF THE INVENTION

Cosmetic compositions used to make up or enhance the appearance of a user's skin are often required to be able to impart various properties such as long wear, transfer resistance and comfort. However, the formulation of cosmetic products that can deliver these properties at the same time can pose some challenges. For example, cosmetic compositions using traditional ingredients known to impart long wear, such as silicone resins, are very drying. In addition, they cause discomfort and flaking during the use. In order to overcome these problems, oils, such as silicone oils are generally employed. US 2007/053859 discloses compositions containing at least one polyorganosiloxane-containing polymer, at least one volatile non-silicone oil, at least one alkylene polymer wax, at least one silicone film-forming agent and, optionally, at least one long-chain alcohol wax, at least one coloring agent, and/or at least one volatile silicone oil, as well as to methods for using such compositions and to kits containing such compositions. While the utilization of silicone oils in cosmetics is popular, one drawback associated with their use is that they tend to shine and are tacky, which are not always desired effects for the finished products.

Therefore, it is an object of the present invention to provide a composition and method for making up skin in a manner which delivers transfer resistance and long wear properties, as well as superior comfort, non-tacky feel and nonglossy (matte) appearance.

It has been surprisingly discovered that the combination of silicone crosspolymer (elastomer) and silicone oil (fluid) having viscosity greater than 100 cSt at specific ratios, in addition to silicone resins, polyorganosiloxane copolymer and volatile solvent, provides the compositions characterized by non-tacky feel and superior comfort, transfer resistance, long wear and minimal or absence of flaking when applied onto a keratinous substrate. The formulations of the present inventions are also matte. In this context, the document US2007053859A1 is relevant.

### BRIEF SUMMARY OF THE INVENTION

According to one embodiment of the present invention, there is provided a composition as defined in claim 1, preferably anhydrous, which is long wearing and transfer resistant, while at the same time provides superior comfort, non-tacky feel and looks matte, containing:
(a) from 2% to 10% by weight of at least one silicone elastomer;
(b) from about 2% to about 30% by weight of at least one non-volatile oil, wherein the at least one non-volatile oil is a dimethicone fluid having a viscosity greater than 0.0003 m²/s (300 cSt) and lower than 0.0012 m²/s (1200 cSt) at 25°C;
(c) from 2% to 35% by weight of polyorganosiloxane copolymer;
(d) from 5% to 30% by weight of at least one silicone resin;
(e) from 5% or 50% of at least one volatile solvent, wherein the volatile solvent is a hydrocarbon oil having from 8 to 16 carbon atoms or a silicone oil having a viscosity, at 25 °C, of less than or equal to 0.000006 m²/s (6 cSt), and having from 2 to 7 silicon atoms, optionally substituted with alkyl or alkoxy groups of 1 to 10 carbon atoms, and their mixtures;
(f) optionally at least one wax;
(g) optionally at least one colorant; and
(h) optionally at least one filler;
wherein the weight_ratio of the silicone elastomer (a) to the at least one non-volatile oil (b) is from 1:0.02 to 1:10; the weights being relative to the total weight of the composition.

Thus, the ratio of the silicone elastomer (a) to the at least one non-volatile oil (b) is higher or equals to from about 1:0.02 (e.g. is from about 1:1 to about 1:6) and is lower or equals from about 1:10, by weight, the weights being relative to the total weight of the composition. All numerical values are weight percent solids (actives).

According to a preferred embodiment, the composition further contains at least one colorant, at least one wax, at least one filler and at least one additive.

As per this invention, the inventive compositions are related but not limited to liquid compositions, such as lipsticks, liners, foundations, mascaras, eyeshadows, skin care compositions, sunscreens, skin repellants, deodorants, nail composition. For example, there is provided a lipstick liquid composition as defined in claim 13 and a method as defined in claim 14 of making up lips comprising applying to the lips a lipstick composition.

Another embodiment of this invention pertains to the composition being free or substantially free or devoid of non-volatile solvents having at least one or more phenyl groups. These solvents are for example described in US. Pat. No. 8, 945,525.

In another embodiment, the invention is a method of making up skin involving applying onto the skin the above disclosed compositions, as well as a method of making the inventive composition.

As per another embodiment, this invention relates to a system of cosmetic compositions comprising the color coat composition as previously described and a top coat composition.

According to the invention, the term "anhydrous" refers to a composition not containing any water, that is to say a composition in which the water that may be present comes only from the water of crystallization or of adsorption of the starting materials. In any case, an anhydrous composition contains less than 5% by weight of water, preferably less than 1% by weight, and better still less than 0.5% by weight of water, relative to the total weight of the composition.

As used herein, the expression "at least one" means one or more and thus includes individual components as well as mixtures/combinations.

Other than in the operating examples, or where otherwise indicated, all numbers expressing quantities of ingredients and/or reaction conditions are to be understood as being modified in all instances by the term "about," meaning within 10% to 15% of the indicated number (e.g. "about 10%" means 9% - 11% and "about 2%" means 1.8% - 2.2%).

"Film former" or "film forming agent" or "film forming polymer" or "film forming resin" as used herein mean a polymer or resin that leaves a film on the substrate to which it is applied, for example, after a solvent accompanying the film former has evaporated, absorbed into and/or dissipated on the substrate.

"Transfer resistance" as used herein refers to the quality exhibited by compositions that are not readily removed by contact with another material, such as, for example, a glass, an item of clothing or the skin, for example, when eating or drinking. Transfer resistance may be evaluated by any method known in the art for evaluating such. For example, transfer resistance of a composition may be evaluated by a "kiss" test. The "kiss" test may involve application of the composition to human keratin material such as hair, skin or lips followed by rubbing a material, for example, a sheet of paper, against the hair, skin or lips after expiration of a certain amount of time following application, such as 2 minutes after application. Similarly, transfer resistance of a composition may be evaluated by the amount of product transferred from a wearer to any other substrate, such as transfer from the hair, skin or lips of an individual to a collar when putting on clothing after the expiration of a certain amount of time following application of the composition to the hair, skin or lips. The amount of composition transferred to the substrate (e.g., collar, or paper) may then be evaluated and compared. For example, a composition may be transfer resistant if a majority of the product is left on the wearer's hair, skin or lips. Further, the amount transferred may be compared with that transferred by other compositions, such as commercially available compositions. In a preferred embodiment of the present invention, little or no composition is transferred to the substrate from the hair, skin or lips.

"Long wear" compositions as used herein, refers to compositions where color remains the same or substantially the same as at the time of application, as viewed by the naked eye, after an extended period of time. Long wear properties may be evaluated by any method known in the art for evaluating such properties. For example, long wear may be evaluated by a test involving the application of a composition to human hair, skin or lips and evaluating the color of the composition after an extended period of time. For example, the color of a composition may be evaluated immediately following application to hair, skin or lips and these characteristics may then be re-evaluated and compared after a certain amount of time. Further, these characteristics may be evaluated with respect to other compositions, such as commercially available compositions. For lip compositions, "long wear" typically means the composition remains on the lips at least about 4 hours up to about 24 hours, and retains rich color even after eating.

"Liquid" or "liquid cosmetic" or "liquid lipstick" or "liquoid composition" means a composition having a fixed volume, flows to cover the bottom and assumes the shape of the portion of the container it fills and is slightly compressible (as disclosed in General chemistry, Fourth Edition 2005, p.434

"Tackiness" as used herein refers to the adhesion between two substances. For example, the more tackiness there is between two substances, the more adhesion there is between the substances. To quantify "tackiness," it is useful to determine the "work of adhesion" as defined by IUPAC associated with the two substances. Generally speaking, the work of adhesion measures the amount of work necessary to separate two substances. Thus, the greater the work of adhesion associated with two substances, the greater the adhesion there is between the substances, meaning the greater the tackiness is between the two substances.

Work of adhesion and, thus, tackiness, can be quantified using acceptable techniques and methods generally used to measure adhesion and the one far along described.

"Substituted" as used herein, means comprising at least one substituent. Non-limiting examples of substituents include atoms, such as oxygen atoms and nitrogen atoms, as well as functional groups, such as amine groups, ether groups, alkoxy groups, acyloxyalky groups, oxyalkylene groups, polyoxyalkylene groups, carboxylic acid groups, amine groups, acylamino groups, amide groups, halogen containing groups, ester groups, thiol groups, sulphonate groups, thiosulphategroups, siloxane groups, and polysiloxane groups. The substituent(s) may be further substituted.

"Comprising" it is meant that other steps and/or ingredients which do not affect the end result may be added. The products, compositions, methods and processes of the present invention can include all the essential elements and limitations of the invention described herein as well as any of the additional or optional ingredients, components, steps, or limitations described herein.

"Free" or "substantially free" or "devoid of" as it is used herein means that while it is preferred that no amount of the specific component be present in the composition, it is possible to have very small amounts of it in the compositions of the invention provided that these amounts do not materially affect at least one, preferably most, of the advantageous properties of the conditioning compositions of the invention. Thus, for example, "free of phenylated solvents" means that non-volatile solvents are preferably omitted (that is 0% by weight), but can be present in the composition at an amount of less than about 0.25% by weight, typically less than about 0.1% by weight, typically less than about 0.05% by weight, based on the total weight of the composition as a whole.

As used herein, all ranges provided are meant to include every specific range within, and combination of subranges between, the given ranges. Thus, a range from 1-5, includes specifically 1, 2, 3, 4 and 5, as well as subranges such as and 2-5, 3-5, 2-3, 2-4, 1-4, etc.

As used herein a range of ratios is meant to include every specific ratio within, and combination of subranges between the given ranges.

The compositions and methods of the present invention can comprise, consist of, or consist essentially of the essential elements and limitations of the invention described herein, as well as any additional or optional ingredients, components, or limitations described herein or otherwise useful.

### Siloxysilicate Resins

The cosmetic compositions of the present invention comprise at least one silicone resin such as described for example, in U.S. Pat. No. 5,505,937, U.S. Pat. No. 5,911,974, U.S. Pat. No. 5,965,112, U.S. Pat. No. 5,985,298, U.S. Pat. No. 6,074,654, U.S. Pat. No. 6,780,422, U.S. Pat. No. 6,908,621.

According to this invention, the cosmetic compositions may contain siloxysilicate resins. One non-limiting example of a siloxysilicate in accordance with the present invention is trimethylsiloxysilicate, which may be represented by the following formula:

[(CH₃)₃SiO]ₓ(SiO_{4/2})_{y}

wherein x and y may, for example, range from 50 to 80. Such siloxysilicates are commercially available from General Electric, Dow Corning, Wacker, Milliken, Siltech, Grant Industries, Momentive and Shin-Etsu Silicones under the tradename Resin MQ^{®}.

According to another embodiment of this invention, the compositions may contain silsesquioxane resins, including comprise at least one polypropyl silsesquioxane film forming resin.

Silsesquioxane resins are a specific form of silicone resin. Silicone resin nomenclature is known in the art as "MDTQ" nomenclature, whereby a silicone resin is described according to the various monomeric siloxane units which make up the polymer. Each letter of "MDTQ" denotes a different type of unit. When the film forming resin is made up predominantly of tri-functional units (or T units), it is generally called a silsesquioxane resin, which is described, for example in US 2006/0292096.

Examples of silsesquioxane resins that may be used in the present invention are alkyl silsesquioxane resins that are silsesquioxane homopolymers and/or copolymers having an average siloxane unit of the general formula R¹ₙ SiO_{(4-n)/2}, wherein each R1 is a propyl group, wherein more than 80 mole % of R1 represent a C3 -C10 alkyl group, n is a value of from 1.0 to 1.4, and more than 60 mole % of the copolymer comprises R¹SiO_{3/2} units. As each R1 is a propyl group these polymers are called polypropylsilsesquioxane resins or "t-propyl" silsesquioxane resins. These resins and methods of making them are described, for example in US 8,586,013, 2012/0301415, 2007/0093619, and 2006/0292096.

A non-limiting example of a polypropylsilsesquioxane resin suitable for use in the present invention is commercially available from Dow Corning as Dow Corning 670 Fluid or Dow Corning 680 Fluid. These Dow Corning resins have a general formula of RₙSiO_{(4-n)/2} wherein R is independently chosen from a hydrogen atom and a monovalent hydrocarbon group comprising 3 carbon atoms, wherein more than 80 mole % of R are propyl groups, n is a value from 1.0 to 1.4, more than 60 mole % of the copolymer comprises RSiO_{3/2} units, and having a hydroxyl or alkoxy content from 0.2 to 10% by weight, for example between 1 and 4% by weight, preferably between 5 and 10% by weight, and more preferably between 6 and 8% by weight. Preferably, the polypropylsilsesquioxane resin has a molecular weight from about 5000 to about 30,000 and a Tg from about -5°C to about 5°C.

Another embodiment of this invention, exemplifies the composition containing at least one siloxysilicate resin, at least one silsesquioxane resin and/or mixture thereof.

The at least one silicone resin is present in the cosmetic composition of the present invention in an amount ranging from 5% to 30% by weight; such as from about 10% to about 25% by weight; such as from about 15% to about 20% by weight, all weights being based on the weight of the composition as a whole.

### Polyorganosiloxane Copolymer

The cosmetic compositions of the present invention also comprise at least one polyorganosiloxane-containing polymer. The polyorganosiloxane-containing polymer useful herein is a polymer (homopolymer or copolymer) having at least one moiety which contains: at least one polyorganosiloxane group consisting of 1 to about 1000 organosiloxane units in the chain of the moiety or in the form of a graft, and at least two groups capable of establishing hydrogen interactions. Non-limiting examples of polyorganosiloxane-containing polymers are disclosed, for example in US. Pat. No. 8,945,525.

Additional polyorganosiloxane-containing polymers which may be used in the composition of the invention include those described in documents US5,874,069, US5,919,441, US6,051,216, and US5,981,680.

A preferred polyorganosiloxane-containing polymer for use in the present invention contain at least one moiety chosen from formula (III): 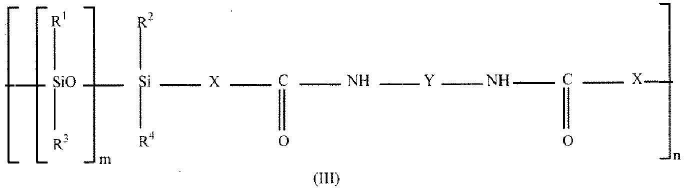 and formula (IV) 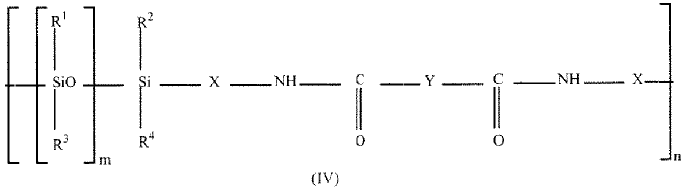 in which:
(a) R¹, R², R³ and R⁴ are the same or different and may be selected from the group consisting of methyl, ethyl, propyl, isopropyl, a siloxane chain, and phenyl;
(b) X is a linear or branched chain alkylene having 1-30 carbons;
(c) Y is selected from the group consisting of linear or branched chain alkylenes having 1-40 carbons;
(d) m is a number between 1 and 700;
(e) n is a number between 1 and 500.

Particularly preferred polyorganosiloxane-containing polymers useful herein are commercially available from Dow Corning under the tradenames DC 8178^{®} and DC 8179^{®}, which are known under the INCI denomination of Nylon-611/Dimethicone Copolymer.

The at least one polyorganosiloxane-containing polymer is present in the cosmetic composition of the present invention in an amount ranging from 2% to 35% by weight; such as from about 5% to about 30% by weight; such as from about 7% to about 20% by weight, all weights being based on the weight of the composition as a whole.

### Silicone Elastomer (silicone crosspolymer)

The composition according to the invention also comprises at least a silicone elastomer.

In a preferred embodiment, the composition comprises a non-emulsifying silicon elastomer.

The non-emulsifying silicon elastomer may be in the form of a gel or a powder.

The "organopolysiloxane elastomer" or "silicon elastomer" or "silicone crosspolymer" thickens the composition, adds the cushiony (spongy) effect and to improves the application of the finished product. Also, it provides a very soft feel and mattifying effect after the application, which is especially advantageous for skin products.

The term "non-emulsifying" defines organopolysiloxane elastomers that do not contain in any hydrophilic chains, and in particular polyoxyalkylene (especially polyoxyethylene or polyoxypropylene) or polyglyceryl units. Thus, according to one particular embodiment of the invention, the composition comprises an organopolysiloxane elastomer that is free of polyoxyalkylene units and polyglyceryl units.

The non-emulsifying elastomers are described in US. Pat. NO. 8,637,057.

The non-emulsifying elastomers particularly useful in this invention include but not limit those sold under the names KSG-6, KSG-15, KSG-16, KSG-18, KSG-41, KSG-42, KSG-43 and KSG-44 by the company Shin-Etsu, DC 9040, DC 9041, DC 9509, DC 9505 and DC 9506, by the company Dow Corning, and SFE 839 by the company General Electric.

In an embodiment, the organopolysiloxane elastomer particles are conveyed in the form of a gel formed from an elastomeric organopolysiloxane included in at least one hydrocarbon-based oil and/or one silicone oil. In these gels, the organopolysiloxane particles are often non-spherical particles.

Not limited examples of silicone elastomers useful in this invention are dimethicone crosspolymer gels (blends of dimethicone crosspolymers in solvents) having viscosity values from about 150 and to about 700 mm²/s, from about 200 to about 650 mm²/s and from about 300 to about 600 mm²/s.

Particularly useful for this invention may be blends of high molecular weight silicone elastomers in volatile solvents, such as silicone oils, hydrocarbon oils and mixtures thereof, as per definition disclosed far along.

The specific but not limiting examples of silicone elastomeric gels applicable in this invention are represented by **DC EL-8040 ID (INCI name: Isododecane (and) Dimethicone Crosspolymer) and DC** EL-9140 DM (INCI name: Dimethicone (and) Dimethicone Crosspolymer), supplied by Dow Corning.

Non-limiting examples of silicone elastomers and their synthesis are disclosed, for example in U.S. Pat. No. 8, 637,057 and US/20150174048.

This silicon elastomers present in the inventive compositions are in a content ranging from 2 percent to 10 percent by weight relative to the total weight of said composition.

### Volatile Solvent

The compositions of the invention contain at least one volatile solvent, wherein the volatile solvent is a hydrocarbon oil having from 8 to 16 carbon atoms or a silicone oil having a viscosity, at 25 °C, of less than or equal to 0.000006 m²/s (6 cSt), and having from 2 to 7 silicon atoms, optionally substituted with alkyl or alkoxy groups of 1 to 10 carbon atoms, and their mixtures.

The expression "volatile solvent" means a non-aqueous medium capable of evaporating on contact with the skin or the lips in less than one hour at room temperature and atmospheric pressure.

Examples of suitable volatile solvents include volatile hydrocarbon-based oils having from 8 to 16 carbon atoms and their mixtures and in particular branched C₈ to C₁₆ alkanes such as C₈ to C₁₆ isoalkanes (also known as isoparaffins), isododecane, isodecane, isohexadecane, and for example, the oils sold under the trade names of Isopar or Permethyl, the C₈ to C₁₆ branched esters such as isohexyl or isodecyl neopentanoate, alcohols, and their mixtures. Preferably, the volatile hydrocarbon-based oils have a flash point of at least 40°C.

Examples of volatile hydrocarbon-based oils include, but are not limited to those given in Table 1 below.

**Table 1**

| Compound | Flash Point (°C) |
|---|---|
| Isododecane | 43 |
| Isohexadecane | 102 |
| Isodecyl neopentanoate | 118 |
| Isopar L (isoparaffin C₁₁-C₁₃) | 62 |
| Isopar H (isoparaffin C₁₁-C₁₂) | 56 |

The volatile solvent may also be chosen from volatile silicone oils, which may be linear or cyclic, having a viscosity, at room temperature, of less than or equal to 0.000006 m²/s (6 cSt), and having from 2 to 7 silicon atoms, optionally substituted with alkyl or alkoxy groups of 1 to 10 carbon atoms.

Examples of suitable volatile silicone oils include, but are not limited to, those listed in Table 2 below.

**Table 2**

| Compound | Flash Point (°C) | Viscosity (m2/s (cSt)) |
|---|---|---|
| Octyltrimethicone | 93 | 0.0000012 (1.2) |
| Hexyltrimethicone | 79 | 0.0000012 (1.2) |
| Decamethylcyclopentasiloxane (cyclopentasiloxane or D5) | 72 | 0.0000042 (4.2) |
| Octamethylcyclotetrasiloxane (cyclotetradimethylsiloxane or D4) | 55 | 0.0000025 (2.5) |
| Decamethyltetrasiloxane(L4) | 63 | 0.0000017 (1.7) |
| KF-96 A from Shin Etsu | 94 | 0.000006 (6) |
| PDMS (polydimethylsiloxane) DC 200 (1.5cSt) from Dow Corning | 56 | 0.0000015 (1.5) |
| PDMS DC 200 (2cSt) from Dow Corning | 87 | 0.000002 (2) |
| PDMS DC 200 (5cSt) from Dow Corning | 134 | 0.000005 (5) |
| PDMS DC 200 (3St) from Dow Corning | 102 | 0.000003 (3) |

The at least one volatile solvent is present in the cosmetic composition of the present invention in an amount ranging from 5% to 50% by weight; such as from about 10% to about 45% by weight; such as from about 15% to about 40% by weight, all weights being based on the weight of the composition as a whole.

### Non-volatile Solvent

The compositions of the present invention also comprise at least one non-volatile solvent (oil) ), wherein the at least one non-volatile oil is a dimethicone fluid having a viscosity greater than 0.0003 m2/s (300 cSt) and lower than 0.0012 m2/s (1200 cSt) at 25°C. Provided is also a lipstick liquid composition and a method of making up lips comprising applying to the lips a lipstick composition, wherein at least one non-volatile oil is a dimethicone fluid having a viscosity greater than 0.0003 m2/s (300 cSt) and lower than 0.0015 m2/s (1500 cSt) at 25°C.

The volatility of the oils can be determined using the evaporation speed as set forth in U.S. Pat. No. 6,338,839.

In contrast to waxes, oils are liquids at room temperature.

"High viscosity" means an oil having a viscosity greater than 0.0001 m²/s (100 cSt), particularly greater than 0.00025 m²/s (250 cSt) at 25°C. Thus, the dimethicone fluid in the present invention is a high viscosity non-volatile oil. Non-volatile silicone oils are described, for example in US 2011/0293550 and US 2004/0126350.

Particularly useful dimethione fluids have viscosity from about 0.00035 m²/s (350) cPs at 25°C to about 0.001 m²/s (1000 cPs) at 25°C.

Specific examples of suitable for this invention high viscosity silicone oils include, but are not limited to, Xiameter^{®} silicone fluids from Dow Corning.

The at least one non-volatile silicone oil is present in the compositions of the present invention in an amount ranging from about 2% to about 30% by weight, including from about 4% to about 25% by weight, typically about 6% to about 20% by weight, based on the total weight of the composition, including all ranges and subranges within these ranges.

It has been surprisingly discovered that by combining in the inventive composition, at least one silicone elastomer, preferably at least one dimethicone crosspolymer, and at least one non-volatile dimethicone fluid having a viscosity greater than or equal to 0.0003 m²/s (300 cSt) and lower than 0.0012 m²/s (1200 cSt) [or in certain embodiments related to lipstick liquid composition, greater than 0.0003 m2/s (300 cSt) and lower than 0.0015 m2/s (1500 cSt)] at 25°C, in a ratio where the weight of the silicone elastomer to the weight of the dimethicone fluid is higher or equal to from 1:0.02 and is lower or equals to from 1:10 by weight (for example, 1:0.02 to 1:10), including all ranges and subranges therebetween such as, for example, from about 1:1 to about 1:6 and from about 1:1 to about 1:5, yield a cosmetic characterized by long wear, transfer resistance, non-tackiness, limited flaking and great comfort.

According to other embodiments, the present invention refers to methods of improving the tackiness, transfer-resistance and/or long wear properties of a composition, comprising incorporating to the composition, at least one silicone elastomer, preferably at least one dimethicone crosspolymer, and at least one non-volatile dimethicone fluid having a viscosity greater than or equal to 0.0003 m²/s (300 cSt) and lower than 0.0012 m²/s (1200 cSt) [or in certain embodiments related to lipstick liquid composition, greater than 0.0003 m2/s (300 cSt) and lower than 0.0015 m2/s (1500 cSt)] at 25°C, in a ratio where the weight of the silicone elastomer to the weight of the non-volatile dimethicone fluid is higher or equal to from 1:0.02 and is lower or equals to from 1: 10 by weight (for example, 1:0.02 to 1:10), including all ranges and subranges therebetween such as, for example, from about 1:1 to about 1:6 and from about 1:1 to about 1:5, yield a cosmetic characterized by long wear, transfer resistance, non-tackiness, limited flaking and great comfort.

The compositions of the present invention are useful as compositions for making up the skin, in particular the lips.

### Wax (optional)

The cosmetic compositions of the present invention optionally may contain at least one wax.

For the purposes of the present invention, a wax is a lipophilic fatty compound that is solid at room temperature (25°C), has a reversible solid/liquid change of state (that is, the state of the material may change based on temperature), has a melting point greater than 45°C, preferably greater than 55°C, more preferably between about 65°C to about 120°C, and has anisotropic crystal organization in the solid state. The melting point of the wax may be measured using a differential scanning calorimeter (DSC), for example the calorimeter sold under the name DSC 30 by Mettler. For waxes that are derived from petroleum, such as microcrystalline wax, the melting point may be measured according to the drop ASTM method, D-127.

The waxes are those generally used in cosmetics and dermatology. The waxes may be of natural origin, for instance beeswax, carnauba wax, candelilla wax, ouricoury wax, Japan wax, cork fiber wax or sugar cane wax, paraffin wax, lignite wax, microcrystalline waxes, lanolin wax, montan wax, ozokerites and hydrogenated oils, for instance hydrogenated jojoba oil.

The waxes also may be of synthetic origin, for instance polyethylene waxes derived from the polymerization of ethylene, waxes obtained by Fischer-Tropsch synthesis, esters of fatty acids and of glycerides that are solid at 40°C.

Waxes of synthetic origin are preferable as they are more uniform and provide greater reproducibility than waxes of natural origin. Moreover, the waxes are preferably not silicone waxes.

Particular waxes include polyethylene waxes, for example the product sold under the name Performalene 500-L Polyethylene (New Phase Technology), and polymethylene waxes, for instance the product sold under the name Cirebelle 303 (Sasol).

The cosmetic compositions of the present invention may contain at least one polypropylsilsesquioxane wax substituted with alkyl units having at least 30 carbons.

Polypropylsilsesquioxane waxes, in general, have been disclosed in patent publication WO2005/100444 and U.S. Pat. No.8,586,013.

It should be noted, however, that not all polypropylsilsesquioxane waxes yield stable colored cosmetic emulsion products. More particularly, it has been found that only those polypropylsilsesquioxane waxes substituted with alkyl units having at least 30 carbons are stable.

A particularly preferred polypropylsilsesquioxane wax for use in the present invention is a C30-45 ALKYLDIMETHYLSILYL POLYPROPYLSILSESQUIOXANE commercially available from DOW CORNING under the tradename SW-8005 C30 Resin Wax.

When present in the instant compositions, the at least one wax may be present in an amount ranging from about 0.01% to about 30% by weight, from about 0.02% to about 25%, typically from about 0.03% to 15% by weight, preferably from about 0.05% to about 5% by weight, including all ranges and subranges therebetween, all weights based on the weight of the composition as a whole.

### Pigments

The cosmetic compositions of the present invention may also contain at least one cosmetically acceptable colorant such as a pigment or dyestuff. Examples of suitable pigments include, but are not limited to, inorganic pigments, organic pigments, lakes, pearlescent pigments, iridescent or optically variable pigments, and mixtures thereof. A pigment should be understood to mean inorganic or organic, white or colored particles. Said pigments may optionally be surface-treated within the scope of the present invention but are not limited to treatments such as silicones, perfluorinated compounds, lecithin, and amino acids.

Representative examples of inorganic pigments useful in the present invention include those selected from the group consisting of rutile or anatase titanium dioxide, coded in the Color Index under the reference CI 77,891; black, yellow, red and brown iron oxides, coded under references CI 77,499, 77, 492 and, 77,491; manganese violet (CI 77,742); ultramarine blue (CI 77,007); chromium oxide (CI 77,288); chromium hydrate (CI 77,289); and ferric blue (CI 77,510) and mixtures thereof.

Representative examples of organic pigments and lakes useful in the present invention include, but are not limited to, D&C Red No. 19 (CI 45,170), D&C Red No. 9 (CI 15,585), D&C Red No. 21 (CI 45,380), D&C Orange No. 4 (CI 15,510), D&C Orange No. 5 (CI 45,370), D&C Red No. 27 (CI 45,410), D&C Red No. 13 (CI 15,630), D&C Red No. 7 (CI 15,850), D&C Red No. 6 (CI 15,850), D&C Yellow No. 5 (CI 19,140), D&C Red No. 36 (CI 12,085), D&C Orange No. 10 (CI 45,425), D&C Yellow No. 6 (CI 15,985), D&C Red No. 30 (CI 73,360), D&C Red No.3 (CI 45,430) and the dye or lakes based on cochineal carmine (CI 75,570) and mixtures thereof.

Representative examples of pearlescent pigments useful in the present invention include those selected from the group consisting of the white pearlescent pigments such as mica coated with titanium oxide, mica coated with titanium dioxide, bismuth oxychloride, titanium oxychloride, colored pearlescent pigments such as titanium mica with iron oxides, titanium mica with ferric blue, chromium oxide and the like, titanium mica with an organic pigment of the above-mentioned type as well as those based on bismuth oxychloride and mixtures thereof.

The precise amount and type of colorant employed in the compositions of the present invention will depend on the color, intensity and use of the cosmetic composition and, as a result, will be determined by those skilled in the art of cosmetic formulation. However, one preferred amount of colorant for use in the present invention is from about 0.5% to about 15%, typically from about 1.5% to about 12%, most typically from about 2% to about 10%, based on the weight of the composition.

### Filler

Fillers that may be used in the compositions of the invention include, for example, silica powder; talc; polyamide particles and especially those sold under the name Orgasol by the company Atochem; polyethylene powders; microspheres based on acrylic copolymers, such as those based on ethylene glycol dimethacrylate/lauryl methacrylate copolymer sold by the company Dow Corning under the name Polytrap; expanded powders such as hollow microspheres and especially the microspheres sold under the name Expancel by the company Kemanord Plast or under the name Micropearl F 80 ED by the company Matsumoto; powders of natural organic materials such as crosslinked or noncrosslinked corn starch, wheat starch or rice starch, such as the powders of starch crosslinked with octenyl succinate anhydride, sold under the name Dry-Flo by the company National Starch; silicone resin microbeads such as those sold under the name Tospearl by the company Toshiba Silicone; clays (bentone, laponite, saponite, etc.); and mixtures thereof.

The fillers may be present in the composition of the invention in an amount ranging from about 0.1% to about 50% by weight, such as from 0.5% to about 30% by weight, and such as from about 1% to about 20% by weight, all weights based on the weight of the composition as a whole.

As per this invention hydrophobic silica aerogels are particularly useful.

Silica aerogels are porous materials obtained by replacing (by drying) the liquid component of a silica gel with air. They are generally synthesized via a sol-gel process in liquid medium and then dried, usually by extraction of a supercritical fluid, the one most commonly used being supercritical C0₂. This type of drying makes it possible to avoid shrinkage of the pores and of the material. The sol-gel process and the various drying processes are described in detail in Brinker CJ., and Scherer G.W., Sol-Gel Science: New York: Academic Press, 1990. Silica aerogels, in general, have been disclosed in U.S. Pat. No. 9,320,689.

As hydrophobic silica aerogels that may be used in the invention, examples that may be mentioned include the aerogel sold under the name VM-2260 (INCI name: Silica silylate), by the company Dow Corning, the particles of which have a mean size of about 1000 microns and a specific surface area per unit of mass ranging from 600 to 800 m²/g.

Mention may also be made of the aerogels sold by the company Cabot under the references AEROGEL TLD 201, AEROGEL OGD 201, AEROGEL TLD 203, ENOVA^{®} AEROGEL MT 1100, ENOVA AEROGEL MT 1200.

Use will be made more particularly of the aerogel sold under the name VM-2270 (INCI name: Silica silylate), by the company Dow Corning, the particles of which have a mean size ranging from 5-15 microns and a specific surface area per unit of mass ranging from 600 to 800 m²/g.

The silica aerogel particles can be used in the inventive compositions from 0.1% to about 8% by weight, preferably from 0.25% to 6% by weight, better still from 0.5% to 4% by weight, including all ranges and subranges therebetween, all weights based on the weight of the composition as a whole.

### Additives

The compositions of the present invention may further comprise at least one cosmetically or dermatologically acceptable additive such as aa additional thickener, an additional film former, a plasticizer, an antioxidant, an essential oil, a botanical extract, a fragrance, a preserving agent, a fragrance, a pasty fatty substance, a neutralizing agent, and a polymer, and cosmetically active agents and/or dermatological active agents such as, for example, emollients, moisturizers, vitamins, essential fatty acids and medicaments.

As per this inventions, the additives are incorporated from about 0.02% to about 2%, preferably from about 0.1% to about 1.5%, better still from about 0.3% to about 1%.

### EXAMPLES

The present invention will be better understood from the examples which follow. The examples are intended to be nonrestrictive and explanatory only, with the scope of the invention defined by the claims.

### Method of preparation of inventive composition(s)

The mixture of pigment, isododecane and MQ resin was grinded to create a pigment paste. The blend was processed using Disconti Mill until the paste passed the Hegman Gauge test (ASTM D1210-05). Then, the paste grind was added to the remaining ingredients. The mixture was heated to 80 °C and stirred, until a homogeneous liquid composition was obtained. After that, the inventive composition was cooled down to the room temperature and transferred to desired containers and/or applicators.

### Evaluation of inventive compositions: Methods and Test Results

The inventive compositions were tested for tackiness and flaking versus control and comparative compositions. Each of the tested products was tested five (5) times. Testing methods are described below.

### Tack Testing

The films of each formula were deposited onto contrast cards using a 3 MIL drawdown bar and an Automatic Drawdown Machine. The films were dried at room temperature (25 ° C) overnight and analyzed using a Texture Analyzer equipped with a ball probe. Tack force was measured after applying 350 g-force for 10 seconds. Then, the values of the tackiness were correlated to the comfort of wear of the tested products. The samples having the tackiness having values higher than 100 gr/force, were considered to be very uncomfortable to wear. The tack values between 50-100 gr/force, indicated medium comfort, and those with values of less than 50 gr/force were considered to be comfortable.

### Flake Testing

Additionally, the samples were tested for flake resistant properties. The samples of all tested compositions were deposited onto the surface of Thera-Band^{®} intermediate resistance exercise band (7 x 5 cm), using a 3 MIL drawdown bar. The samples were allowed to dry for 4 hours at room temperature (25 ° C) and then they were stretched to the length of 30 cm. The stretching was repeated ten (10) times for each of the samples. During the stretching process, flacking of the dry samples was observed and correlated with durability of wear of the tested products. To define the degree of samples' flacking, the following four (4) point grading scale was used: 0 - no flaking (very good wear), 1 - low flaking (good wear), 2 - medium flaking (medium/acceptable wear) and 3- high flaking (no wear).

**Table 1- Lip Compositions containing DC 8040 silicone elastomer (18% active)**

| Inventive compositions of the liquid lip products containing DC 8040 Silicone elastomer and 1000cSt Dimethicone are represented but not limited by examples in Table 1, as shown below. | | | | | | | | | |
|---|---|---|---|---|---|---|---|---|---|
| **Ingredients/Test results** | **Inventive Compositions (% weight)** | | | | | | | **Control Compositions (% weight)** | |
| | 1 | 2 | 3 | 4 | 5 | 6 | 7 | 1 | 2 |
| MQ | 17.55 | 17.55 | 17.55 | 17.55 | 17.55 | 17.55 | 17.55 | 17.55 | 17.55 |
| PSPA | 9.78 | 9.78 | 9.78 | 9.78 | 9.78 | 9.78 | 9.78 | 9.78 | 9.78 |
| 1000cst Dimethicone fluid | 12.21 | 12.21 | 12.21 | 6.65 | 6.65 | 9.98 | 9.98 | 0 | 0 |
| Pigment blend | 6.41 | 6.41 | 6.41 | 6.41 | 6.41 | 6.41 | 6.41 | 6.41 | 6.41 |
| Silicone resin wax | 0.5 | 0.5 | 0.5 | 0.5 | 0.5 | 0.5 | 0.5 | 0.5 | 0.5 |
| 8040 Silicone elastomer *(18% active) | 25 (4.5%) | 13.89 (2.5%) | 36.11 (6.5%) | 25 (4.5%) | 13.89 (6.5%) | 25 (4.5%) | 36.11 (6.5%) | 25 (4.5%) | 13.89 (6.5%) |
| Fillers and Lauryl lysine | 4.83 | 4.83 | 4.83 | 4.83 | 4.83 | 4.83 | 4.83 | 4.83 | 4.83 |
| Fragrance | 0.1 | 0.1 | 0.1 | 0.1 | 0.1 | 0.1 | 0.1 | 0.1 | 0.1 |
| Isododecane | QS | QS | QS | QS | QS | QS | QS | QS | QS |
| Ratio of silicone elastomer .( % active) : dimethicone | 1:2.7 | 1:4.9 | 1:1.9 | 1:1.5 | 1:1.02 | 1:2.2 | 1:1.5 | - | - |
| Tack 1 | 8.5 | 5.3 | 9.8 | 14 | 12.1 | 7.5 | 34.8 | 77.7 | 81.3 |
| Tack 2 | 6.1 | 6.4 | 10.1 | 18.1 | 13.2 | 6.7 | 7.1 | 76 | 69.4 |
| Tack 3 | 7 | 5.5 | 7.4 | 15.9 | 13.3 | 8.6 | 29.5 | 63.7 | 82.9 |
| Tack 4 | 7 | 5.5 | 7.1 | 18 | 11.1 | 7 | 11.5 | 59.4 | 75 |
| Tack 5 | 5.8 | 7 | 8.6 | 19.7 | 16.7 | 9.5 | 37 | 58.8 | 88 |
| Average Tack | 6.9 | 5.9 | 8.6 | 17.1 | 13.3 | 7.9 | 24 | 67.1 | 79.3 |
| Stdev | 1.1 | 0.7 | 1.4 | 2.2 | 2.1 | 1.2 | 13.8 | 9.1 | 7.2 |
| Flaking | 1 | 2 | 1 | 0 | 0 | 0 | 0 | 0 | 0 |

| | | | | | | | | | |
|---|---|---|---|---|---|---|---|---|---|
| All numerical values in the above table are weight percent active. *Dow Corning^{®} EL-8040 ID; INCI Name: Isododecane (and) Dimethicone | | | | | | | | | |

It was observed that the inventive compositions having the highest ratio of the silicone elastomer containing 18% of actives to the dimethicone oil was the least tacky indicating a very good comfort and had medium flaking, meaning that that the wear was considered to be good. The inventive formulations having lower ratios of the same silicone elastomer to the dimethicone fluid, were characterized by increased tackiness (decreased comfort) and improved flaking (better wear). The tackiness for the control samples without dimethicone oil, was very high meaning that the comfort of wear was very low, and shown no flakiness.

**Table 2- Lip Compositions containing DC 9140 DM silicone elastomer (14% active)**

| Inventive compositions of the liquid lip compositions containing DC 9140 DM Silicone elastomer and 1000cSt Dimethicone are represented but not limited by examples in Table 2, as shown below. | | | | | | | |
|---|---|---|---|---|---|---|---|
| **Ingredients/Test Results** | **Inventive Compositions (% weight)** | | | | | | **Control Composition (% weight)** |
| | 8 | 9 | 10 | 11 | 12 | 13 | 3 |
| MQ | 17.55 | 17.55 | 17.55 | 17.55 | 17.55 | 17.55 | 17.55 |
| PSPA | 9.78 | 9.78 | 9.78 | 9.78 | 9.78 | 9.78 | 9.78 |
| 1000cst Dimethicone fluid | 12.21 | 12.21 | 11 | 10 | 9 | 10 | 0 |
| Lauryl lysine | 1.5 | 1.5 | 1.5 | 1.5 | 1.5 | 1.5 | 1.5 |
| Pigment blend | 6.41 | 6.41 | 6.41 | 6.41 | 6.41 | 6.41 | 6.41 |
| Silicone resin wax | 0.5 | 0.5 | 0.5 | 0.5 | 0.5 | 0.5 | 0.5 |
| 9140 DM Silicone elastomer *(14% active) | 32.14 (4.5%) | 17.86 (2.5%) | 35.71 (5%) | 35.71 (5%) | 35.71 (5%) | 32.14 (4.5%) | 17.86 (2.5%) |
| Fillers | 3.33 | 3.33 | 3.33 | 3.33 | 3.33 | 3.33 | 3.33 |
| Fragrance | 0.1 | 0.1 | 0.1 | 0.1 | 0.1 | 0.1 | 0.1 |
| Isododecane | QS | QS | QS | QS | QS | QS | QS |
| Ratio of silicone elastomer ( % active) : dimethicone | 1:2.7 | 1:4.9 | 1:2.2 | 1:2 | 1:1.8 | 1:2.2 | - |
| Tack 1 | 6.3 | 6 | 8.9 | 6.3 | 14.7 | 5.6 | 103.6 |
| Tack 2 | 6.8 | 5.5 | 6.8 | 7.4 | 8 | 9.1 | 108.2 |
| Tack 3 | 5.7 | 5.6 | 7.2 | 7 | 10 | 6.9 | 104 |
| Tack 4 | 6 | 6.1 | 7.9 | 7.8 | 11.7 | 7.2 | 89.1 |
| Tack 5 | 8.8 | 5.8 | 8.2 | 8.7 | 9.4 | 10.2 | 74.2 |
| Ave Tack | 6.7 | 5.8 | 7.8 | 7.4 | 10.8 | 7.8 | 95.8 |
| Stdev | 1.2 | 0.3 | 0.8 | 0.9 | 2.6 | 1.8 | 14.1 |
| Flaking | 1 | 0 | 2 | 2 | 2 | 1 | 0 |

| | | | | | | | |
|---|---|---|---|---|---|---|---|
| All numerical values in the above table are weight percent active. *Dow Corning^{®} EL-9140 DM ; INCI Name: Dimethicone (and) Dimethicone Crosspolymer | | | | | | | |

As per results presented in Table 2, the inventive samples having the highest ratio of the silicone elastomer (containing 14% of actives) to the dimethicone oil was characterized by the lowest tackiness and providing a very good comfort. The samples did not flake, meaning that they demonstrate a very good wear. The inventive formulations having lower ratios of the same silicone elastomer to dimethicone, had increased both, tackiness and flaking. The tackiness for the control sample was very high and had no flaking. That indicates that the absence of dimethicone significantly increased discomfort of wear, although the wear appeared to be improved.

Based on the result presented in the tables above, the absence of dimethicone oil significantly increased the tack. In addition, the decrease of the concentration of actives in silicone elastomer blends and the increase of dimethicone oil having high viscosity, caused the reduction of tackiness. As per the provided examples, the inventive lip liquid compositions having ratios of the silicone elastomer actives to dimethicone oil from about 1:0.05 to about 1:5 were considered to be very comfortable to wear with minimal or no flacking (good wear).

**Table 3- Relation between viscosities of dimethicone oils and tackiness and flacking of lip liquid compositions**

| In order to determine the most suitable dimethicone fluid for this invention, three (3) dimethicone oils having different viscosity (at 25°C) were tested for tackiness and flacking: 1000 cSt at, 350 cSt and 100 cSt. | | | | |
|---|---|---|---|---|
| Ingredients/Test | Composition | Composition | Composition | Composition |
| Results | w/1000 cst dimethicone oil | w/350 cst dimethicone oil | w/100 cst dimethicone oil | without dimethicone oil |
| MQ | 17.55 | 17.55 | 17.55 | 17.55 |
| PSPA | 9.78 | 9.78 | 9.78 | 9.78 |
| 1000cst Dimethicone fluid | 12.21 | 0 | 0 | 0 |
| 350cst Dimethicone fluid | 0 | 12.21 | 0 | 0 |
| 100cst Dimethicone fluid | 0 | 0 | 12.21 | 0 |
| Caprylyl methicone | 0 | 0 | 0 | 12.21 |
| Lauryl lysine | 1.5 | 1.5 | 1.5 | 1.5 |
| Pigment blend | 6.41 | 6.41 | 6.41 | 6.41 |
| Silicone resin wax | 0.5 | 0.5 | 0.5 | 0.5 |
| 9140 DM Silicone elastomer* (14% active) | 17.86 | 17.86 | 17.86 | 17.86 |
| Fillers | 3.33 | 3.33 | 3.33 | 3.33 |
| Fragrance | 0.1 | 0.1 | 0.1 | 0.1 |
| Isododecane | QS | QS | QS | QS |
| Tack 1 | 6.0 | 5.1 | 64.0 | 35.3 |
| Tack 2 | 5.5 | 4.6 | 56.4 | 34.7 |
| Tack 3 | 5.6 | 4.8 | 61.8 | 34.9 |
| Tack 4 | 6.1 | 5.2 | 49.5 | 36.2 |
| Tack 5 | 5.8 | 4.6 | 41.2 | 36.8 |
| Ave tack | 5.8 | 4.9 | 54.6 | 35.6 |
| Stdev | 0.3 | 0.3 | 9.3 | 0.9 |
| Flaking | 1 | 0 | 0 | 0 |

| | | | | |
|---|---|---|---|---|
| All numerical values in the above table are weight percent active. *Dow Corning^{®} EL-9140 DM ; INCI Name: Dimethicone (and) Dimethicone Crosspolymer | | | | |

Based on the results in Table 3, the composition which contained dimethcione oil having viscosity of 100 cSt, was the most tacky. It was followed by the composition which did not contain the dimethicone oil at all (including Comparative 3 from table 2), and formulation containing 1000 cSt dimethicone and 350 cSt dimethicone respectively. All tested formulations were characterized by no flaking or minimal flaking.

**Table 4- Evaluation of tackiness and flacking of commercial lip compositions**

| Test Results | Comparator A | Comparator B | Comparator C |
|---|---|---|---|
| Tack1 | 80.9 | 11.1 | 119.1 |
| Tack2 | 84.4 | 8.2 | 135.0 |
| Tack 3 | 118.6 | 4.2 | 150.1 |
| Tack 4 | 123.1 | 7.4 | 129.3 |
| Tack 5 | 104.5 | 8.5 | 146.5 |
| Average Tack | 102.3 | 7.9 | 136.0 |
| Standard deviation | 19.2 | 2.5 | 12.7 |
| Flaking | 0 | 1 | 1 |

In order to define improvement of the inventive lip liquid compositions, three (3) commercially available long wear and transfer resistant lip products were studied for the flacking and tackiness. The compositions of the tested comparators are shown below, as well as the results of the tests.

**Comparator A: (1 step color coat liquid lipstick):** isododecane, trimethylsiloxysilicate, nylon-611/dimethicone copolymer, disteardimonium hectorite, lauroyl lysine, c30-45 alkyldimethylsilyl polypropylsilsesquioxane, alumina, propylene carbonate, synthetic fluorphlogopite, silica, calcium sodium borosilicate, calcium aluminum borosilicate, polyethylene terephthalate, parfum/fragrance, aluminum hydroxide, acrylates copolymer, benzyl alcohol, dimethicone, paraffin, tin oxide. [+/- may contain/peut contenir mica, ci 77891/titanium dioxide, ci 77491, ci 77492, ci 77499/iron oxides, ci 15850/red 7, ci 15985/yellow 6 lake, ci 45410/red 28 lake, ci 45380/red 22 lake, ci 19140/yellow 5 lake, ci 42090/blue 1 lake, ci 75470/carmine] f.i.l. d41008/5

### Comparator B:

**Base coat (2 step lip liquid products : color coat and liquid top coat):** isododecane, trimethylsiloxysilicate, nylon-611/dimethicone copolymer, disteardimonium hectorite, lauroyl lysine, c30-45 alkyldimethylsilyl polypropylsilsesquioxane, alumina, propylene carbonate, synthetic fluorphlogopite, silica, calcium sodium borosilicate, calcium aluminum borosilicate, polyethylene terephthalate, parfum/fragrance, aluminum hydroxide, acrylates copolymer, benzyl alcohol, dimethicone, paraffin, tin oxide. [+/- may contain/peut contenir mica, ci 77891/titanium dioxide, ci 77491, ci 77492, ci 77499/iron oxides, ci 15850/red 7, ci 15985/yellow 6 lake, ci 45410/red 28 lake, ci 45380/red 22 lake, ci 19140/yellow 5 lake, ci 42090/blue 1 lake, ci 75470/carmine] f.i.l. d41008/5

**Top coat (liquid) : trimethyl pentaphenyl trisiloxane,** is-diglyceryl polyacyladipate-2, ozokerite, cera alba/beeswax/cire dabeille, calcium sodium borosilicate, calcium aluminum borosilicate, tocopheryl acetate, parfum/fragrance. [+/- may contain/peut contenir mica, ci 77891/titanium dioxide, ci 77491/iron oxides]

**Comparator C: (1 step liquid color coat lipstick)** : Isododecane, Dimethicone, Trimethylsiloxysilicate, Polyethylene, Synthetic Fluorphlogopite, Disteardimonium Hectorite, C12-15 Alkyl Benzoate, Propylene Carbonate, Silica, Calcium Sodium Borosilicate, Methicone, Sorbic Acid. May Contain: Mica, Titanium Dioxide (Ci 77891), Iron Oxides (Ci 77491, 77492, 77499), Red 7 Lake (Ci 15850), Red 6 Lake (Ci 15850), Yellow 5 Lake (Ci 19140), Red 33 Lake (Ci 17200), Yellow 6 (Ci 15985), Carmine (Ci 75470).

As per results presented above, commercially available long-wear, transfer resistant products did not provide a good comfort, as indicated by very high tack values (over 100 gr/force). According to the data, Product C (1 step liquid lipstick) had the tackiness values above 100 gram/force, it was followed by product A (also 1 step liquid lipstick). However, the tackiness of Product B (2 step product involving color coat and top coat) was very low and comparable with tackiness of the inventive compositions (Table 1, examples 1-7).

## Claims

1. A cosmetic composition comprising:
(a) from 2% to 10% by weight of at least one silicone elastomer;
(b) from 2% to 30% by weight of at least one non-volatile oil, wherein the at least one non-volatile oil is a dimethicone fluid having a viscosity greater than 0.0003 m²/s (300 cSt) and lower than 0.0012 m²/s (1200 cSt) at 25°C;
(c) from 2% to 35% by weight of at least one polyorganosiloxane copolymer;
(d) from 5% to 30% by weight of at least one silicone resin;
(e) from 5% to 50% of at least one volatile solvent, wherein the volatile solvent is a hydrocarbon oil having from 8 to 16 carbon atoms or a silicone oil having a viscosity, at 25 °C, of less than or equal to 0.000006 m²/s (6 cSt), and having from 2 to 7 silicon atoms, optionally substituted with alkyl or alkoxy groups of 1 to 10 carbon atoms, and their mixtures;
(f) optionally at least one wax;
(g) optionally at least one colorant; and
(h) optionally at least one filler;
wherein the weight_ratio of the silicone elastomer (a) to the at least one non-volatile oil (b) is from 1:0.02 to 1:10; the weights being relative to the total weight of the composition.

2. The composition according to any of the preceding claims, wherein the at least one silicone elastomer is non-emulsifying silicone elastomer.

3. The composition according to any of the preceding claims, wherein the at least one silicone elastomer is at least one dimethicone crosspolymer.

4. The composition according to any of the preceding claims, wherein the dimethicone fluid is present in an amount from 6% to 20% by weight being relative to the total weight of the composition.

5. The composition according to any of the preceding claims, wherein the at least one polyorganosiloxane copolymer is Nylon-611/ Dimethicone copolymer, present in an amount from 7% to 20% by weight, relative to the total weight of the composition.

6. The composition according to any of the preceding claims, wherein the at least one silicone resin is selected from siloxysilicate resin, silsesquioxane resin and mixture thereof.

7. The composition according to any of the preceding claims, wherein the at least one silicone resin is present from 10% to 20% by weight, relative to the total weight of the composition.

8. The composition according to any of the preceding claims, wherein the at least one volatile solvent is present from 15% to 40% by weight, relative to the total weight of the composition.

9. The composition according to any of the preceding claims, wherein the composition further contains at least one colorant in amount from 0.5% to 15% by weight, relative to the total weight of the composition.

10. The composition according to any of the preceding claims, wherein the composition further contains at least one wax in amount from 0.01% to 30% by weight, relative to the total weight of the composition.

11. The composition according to any of the preceding claims, wherein the composition further contains at least one filler in amount from 0.1% to 50% by weight, relative to the total weight of the composition.

12. The composition according to any of the preceding claims wherein the composition comprises phenylated solvents in an amount of less than about 0.25% by weight.

13. A lipstick liquid composition comprising:
a) from 2% to 10% by weight of at least one silicone elastomer;
b) from 2% to 30% by weight of at least one non-volatile oil, wherein the at least one non-volatile oil is a dimethicone fluid having viscosity greater than or equal to 0.0003 m²/s (300 cSt) to 0.0015 m²/s (1500 cSt);
c) from 2% to 35% by weight of Nylon-611/Dimethicone copolymer;
d) from 5% to 30% by weight of at least one siloxysilicate resin;
e) from 5% to 50% of at least one volatile hydrocarbon solvent, wherein the volatile hydrocarbon solvent is a hydrocarbon oil having from 8 to 16 carbon atoms and their mixtures;
f) optionally at least one wax;
g) optionally at least one colorant; and
h) optionally at least one filler;
wherein the weight ratio of the silicone elastomer (a) to the at least one non-volatile oil (b) is from 1:0.02 to 1:10; the weights being relative to the total weight of the composition.

14. A method of making up lips comprising applying to the lips a lipstick composition comprising:
a) from 2% to 10% by weight of at least one silicone elastomer;
b) from 2% to 30% by weight of at least one non-volatile oil, wherein the at least one non-volatile oil is a dimethicone fluid having a viscosity greater than 0.0003 m²/s (300 cSt) and lower than 0.0015 m²/s (1500 cSt) at 25°C;
c) from 2% to 35% by weight of at least one polyorganosiloxane copolymer;
d) from 5% to 30% by weight of at least one silicone resin;
e) from 5% to 50% of at least one volatile solvent, wherein the volatile solvent is a hydrocarbon oil having from 8 to 16 carbon atoms or a silicone oil having a viscosity, at 25 °C, of less than or equal to 0.000006 m²/s (6 cSt), and having from 2 to 7 silicon atoms, optionally substituted with alkyl or alkoxy groups of 1 to 10 carbon atoms, and their mixtures;
f) optionally at least one wax;
g) optionally at least one colorant; and
h) optionally at least one filler;
wherein the weight ratio of the silicone elastomer (a) to the at least one non-volatile oil (b) is from 1:0.2 to 1:10; the weights being relative to the total weight of the composition.

## Patentansprüche

1. Eine kosmetische Zusammensetzung, umfassend:
(a) von 2 bis 10 Gew.-% mindestens eines Silikonelastomers;
(b) von 2 bis 30 Gew.-% mindestens eines nichtflüchtigen Öls, wobei das mindestens eine nichtflüchtige Öl ein Dimethiconfluid ist, das bei 25 °C eine Viskosität von mehr als 0,0003 m²/s (300 cSt) und weniger als 0,0012 m²/s (1200 cSt) aufweist;
(c) von 2 bis 35 Gew.-% mindestens eines Polyorganosiloxan-Copolymers;
(d) von 5 bis 30 Gew.-% mindestens eines Silikonharzes;
(e) von 5 bis 50 % mindestens eines flüchtigen Lösungsmittels, wobei das flüchtige Lösungsmittel ein Kohlenwasserstofföl mit 8 bis 16 Kohlenstoffatomen oder ein Silikonöl mit einer Viskosität bei 25 °C von weniger als oder gleich 0,000006 m²/s (6 cSt) ist, und mit 2 bis 7 Siliciumatomen, die optional durch Alkyl- oder Alkoxygruppen mit 1 bis 10 Kohlenstoffatomen substituiert sind, sowie deren Mischungen;
(f) optional mindestens ein Wachs;
(g) optional mindestens einen Farbstoff; und
(h) optional mindestens einen Füllstoff;
wobei das Gewichtsverhältnis des Silikonelastomers (a) zu dem mindestens einen nichtflüchtigen Öl (b) von 1:0,02 bis 1:10 liegt; wobei sich die Gewichte auf das Gesamtgewicht der Zusammensetzung beziehen.

2. Die Zusammensetzung gemäß einem der vorstehenden Ansprüche, wobei das mindestens eine Silikonelastomer ein nicht emulgierendes Silikonelastomer ist.

3. Die Zusammensetzung gemäß einem der vorstehenden Ansprüche, wobei das mindestens eine Silikonelastomer mindestens ein Dimethicon-Crosspolymer ist.

4. Die Zusammensetzung gemäß einem der vorstehenden Ansprüche, wobei das Dimethiconfluid in einer Menge von 6 bis 20 Gew.-%, bezogen auf das Gesamtgewicht der Zusammensetzung, vorliegt.

5. Die Zusammensetzung gemäß einem der vorstehenden Ansprüche, wobei das mindestens eine Polyorganosiloxan-Copolymer Nylon-611/ Dimethicone Copolymer ist, das in einer Menge von 7 bis 20 Gew.-%, bezogen auf das Gesamtgewicht der Zusammensetzung, vorhanden ist.

6. Die Zusammensetzung gemäß einem der vorstehenden Ansprüche, wobei das mindestens eine Silikonharz ausgewählt ist aus Siloxysilikatharz, Silsesquioxanharz und einer Mischung davon.

7. Die Zusammensetzung gemäß einem der vorstehenden Ansprüche, wobei das mindestens eine Silikonharz in einer Menge von 10 bis 20 Gew.-%, bezogen auf das Gesamtgewicht der Zusammensetzung, vorhanden ist.

8. Die Zusammensetzung gemäß einem der vorstehenden Ansprüche, wobei das mindestens eine flüchtige Lösungsmittel in einer Menge von 15 bis 40 Gew.-%, bezogen auf das Gesamtgewicht der Zusammensetzung, vorhanden ist.

9. Die Zusammensetzung gemäß einem der vorstehenden Ansprüche, wobei die Zusammensetzung ferner mindestens einen Farbstoff in einer Menge von 0,5 bis 15 Gew.-%, bezogen auf das Gesamtgewicht der Zusammensetzung, enthält.

10. Die Zusammensetzung gemäß einem der vorstehenden Ansprüche, wobei die Zusammensetzung ferner mindestens ein Wachs in einer Menge von 0,01 bis 30 Gew.-%, bezogen auf das Gesamtgewicht der Zusammensetzung, enthält.

11. Die Zusammensetzung gemäß einem der vorstehenden Ansprüche, wobei die Zusammensetzung ferner mindestens einen Füllstoff in einer Menge von 0,1 bis 50 Gew.-%, bezogen auf das Gesamtgewicht der Zusammensetzung, enthält.

12. Die Zusammensetzung gemäß einem der vorstehenden Ansprüche, wobei die Zusammensetzung phenylierte Lösungsmittel in einer Menge von weniger als etwa 0,25 Gew.-% umfasst.

13. Eine flüssige Lippenstiftzusammensetzung, umfassend:
(a) von 2 bis 10 Gew.-% mindestens eines Silikonelastomers;
(b) von 2 bis 30 Gew.-% mindestens eines nichtflüchtigen Öls, wobei das mindestens eine nichtflüchtige Öl ein Dimethiconfluid mit einer Viskosität von größer als oder gleich 0,0003 m²/s (300 cSt) bis 0,0015 m²/s (1500 cSt) ist;
(c) von 2 bis 35 Gew.-% von Nylon-611/Dimethicone Copolymer;
(d) von 5 bis 30 Gew.-% mindestens eines Siloxysilikatharzes;
(e) von 5 bis 50 Gew.-% mindestens eines flüchtigen Kohlenwasserstofflösungsmittels, wobei das flüchtige Kohlenwasserstofflösungsmittel ein Kohlenwasserstofföl mit 8 bis 16 Kohlenstoffatomen und deren Mischungen ist;
(f) optional mindestens ein Wachs;
(g) optional mindestens einen Farbstoff; und
(h) optional mindestens einen Füllstoff;
wobei das Gewichtsverhältnis des Silikonelastomers (a) zu dem mindestens einen nichtflüchtigen Öl (b) von 1:0,02 bis 1:10 liegt; wobei sich die Gewichte auf das Gesamtgewicht der Zusammensetzung beziehen.

14. Ein Verfahren zum Schminken der Lippen, umfassend das Auftragen einer Lippenstiftzusammensetzung auf die Lippen, umfassend:
(a) von 2 bis 10 Gew.-% mindestens eines Silikonelastomers;
(b) von 2 bis 30 Gew.-% mindestens eines nichtflüchtigen Öls, wobei das mindestens eine nichtflüchtige Öl ein Dimethiconfluid ist, das bei 25 °C eine Viskosität von mehr als 0,0003 m²/s (300 cSt) und weniger als 0,0015 m²/s (1500 cSt) aufweist;
(c) von 2 bis 35 Gew.-% mindestens eines Polyorganosiloxan-Copolymers;
(d) von 5 bis 30 Gew.-% mindestens eines Silikonharzes;
(e) von 5 bis 50% mindestens eines flüchtigen Lösungsmittels, wobei das flüchtige Lösungsmittel ein Kohlenwasserstofföl mit 8 bis 16 Kohlenstoffatomen oder ein Silikonöl mit einer Viskosität bei 25 °C von weniger als oder gleich 0,000006 m²/s (6 cSt) ist, und mit 2 bis 7 Siliciumatomen, die optional durch Alkyl- oder Alkoxygruppen mit 1 bis 10 Kohlenstoffatomen substituiert sind, sowie deren Mischungen;
(f) optional mindestens ein Wachs;
(g) optional mindestens einen Farbstoff; und
(h) optional mindestens einen Füllstoff;
wobei das Gewichtsverhältnis des Silikonelastomers (a) zu dem mindestens einen nichtflüchtigen Öl (b) von 1:0,2 bis 1:10 liegt; wobei sich die Gewichte auf das Gesamtgewicht der Zusammensetzung beziehen.

## Revendications

1. Une composition cosmétique comprenant :
(a) de 2 % à 10 % en poids d'au moins un élastomère de silicone ;
(b) de 2 % à 30 % en poids d'au moins une huile non volatile, dans laquelle la au moins une huile non volatile est un fluide de diméthicone ayant une viscosité supérieure à 0,0003 m²/s (300 cSt) et inférieure à 0,0012 m²/s (1200 cSt) à 25 °C ;
(c) de 2 % à 35 % en poids d'au moins un copolymère de polyorganosiloxane ;
(d) de 5 % à 30 % en poids d'au moins une résine de silicone ;
(e) de 5 % à 50 % d'au moins un solvant volatil, dans lequel le solvant volatil est une huile hydrocarbonée ayant de 8 à 16 atomes de carbone ou une huile de silicone ayant une viscosité, à 25 °C, inférieure ou égale à 0,000006 m²/s (6 cSt), et ayant de 2 à 7 atomes de silicium, éventuellement substitués par des groupes alkyle ou alcoxy de 1 à 10 atomes de carbone, ainsi que leurs mélanges ;
(f) éventuellement au moins une cire ;
(g) éventuellement au moins un colorant ; et
(h) éventuellement au moins une charge ;
dans laquelle le rapport pondéral entre l'élastomère de silicone (a) et la au moins une huile non volatile (b) est compris entre 1:0,02 et 1:10, les poids étant exprimés par rapport au poids total de la composition.

2. La composition selon l'une quelconque des revendications précédentes, dans laquelle le au moins un élastomère de silicone est un élastomère de silicone non émulsionnant.

3. La composition selon l'une quelconque des revendications précédentes, dans laquelle le au moins un élastomère de silicone est au moins un polymère réticulé à base de diméthicone.

4. La composition selon l'une quelconque des revendications précédentes, dans laquelle le fluide de diméthicone est présent en une quantité de 6 % à 20 % en poids par rapport au poids total de la composition.

5. La composition selon l'une quelconque des revendications précédentes, dans laquelle le au moins un copolymère de polyorganosiloxane est Nylon-611/Dimethicone copolymer, présent en une quantité de 7 % à 20 % en poids, par rapport au poids total de la composition.

6. La composition selon l'une quelconque des revendications précédentes, dans laquelle la au moins une résine de silicone est choisie parmi une résine siloxysilicate, une résine silsesquioxane et un mélange de celles-ci.

7. La composition selon l'une quelconque des revendications précédentes, dans laquelle la au moins une résine de silicone est présente de 10 % à 20 % en poids, par rapport au poids total de la composition.

8. La composition selon l'une quelconque des revendications précédentes, dans laquelle le au moins un solvant volatil est présent de 15 % à 40 % en poids, par rapport au poids total de la composition.

9. La composition selon l'une quelconque des revendications précédentes, dans laquelle la composition contient en outre au moins un colorant en une quantité de 0,5 % à 15 % en poids, par rapport au poids total de la composition.

10. La composition selon l'une quelconque des revendications précédentes, dans laquelle la composition contient en outre au moins une cire en une quantité de 0,01 % à 30 % en poids, par rapport au poids total de la composition.

11. La composition selon l'une quelconque des revendications précédentes, dans laquelle la composition contient en outre au moins une charge en une quantité de 0,1 % à 50 % en poids, par rapport au poids total de la composition.

12. La composition selon l'une quelconque des revendications précédentes, dans laquelle la composition comprend des solvants phénylés en une quantité inférieure à environ 0,25 % en poids.

13. Une composition liquide de rouge à lèvres comprenant :
a) de 2 % à 10 % en poids d'au moins un élastomère de silicone ;
b) de 2 % à 30 % en poids d'au moins une huile non volatile, dans laquelle la au moins une huile non volatile est un fluide de diméthicone ayant une viscosité supérieure ou égale à 0,0003 m²/s (300 cSt) à 0,0015 m²/s (1500 cSt) ;
c) de 2 % à 35 % en poids de Nylon-611/Dimethicone copolymer ;
d) de 5 % à 30 % en poids d'au moins une résine de siloxysilicate ;
e) de 5 % à 50 % d'au moins un solvant hydrocarbure volatile, dans lequel le solvant hydrocarbure volatile est une huile hydrocarbonée ayant de 8 à 16 atomes de carbone et leurs mélanges ;
f) éventuellement au moins une cire ;
g) éventuellement au moins un colorant ; et
h) éventuellement au moins une charge ;
dans laquelle le rapport pondéral entre l'élastomère de silicone (a) et la ou les huiles non volatiles (b) est compris entre 1:0,02 et 1:10, les poids étant exprimés par rapport au poids total de la composition.

14. Une méthode de maquillage des lèvres comprenant l'application sur les lèvres d'une composition de rouge à lèvres comprenant :
a) de 2 % à 10 % en poids d'au moins un élastomère de silicone ;
b) de 2 % à 30 % en poids d'au moins une huile non volatile, dans laquelle la au moins une huile non volatile est un fluide de diméthicone ayant une viscosité supérieure à 0,0003 m²/s (300 cSt) et inférieure à 0,0015 m²/s (1500 cSt) à 25 °C ;
c) de 2 % à 35 % en poids d'au moins un copolymère de polyorganosiloxane ;
d) de 5 % à 30 % en poids d'au moins une résine de silicone ;
e) de 5 % à 50 % d'au moins un solvant volatil, dans lequel le solvant volatil est une huile hydrocarbonée ayant de 8 à 16 atomes de carbone ou une huile de silicone ayant une viscosité, à 25 °C, inférieure ou égale à 0,000006 m²/s (6 cSt), et ayant de 2 à 7 atomes de silicium, éventuellement substitués par des groupes alkyle ou alcoxy de 1 à 10 atomes de carbone, et leurs mélanges ;
f) éventuellement au moins une cire ;
g) éventuellement au moins un colorant ; et
h) éventuellement au moins une charge ;
dans laquelle le rapport pondéral entre l'élastomère de silicone (a) et la ou les huiles non volatiles (b) est compris entre 1:0,2 et 1:10 ; les poids étant exprimés par rapport au poids total de la composition.
